# EUROPEAN PATENT APPLICATION

(11) **EP 1 839 664 A1**
(43) Date of publication of application: **03.10.2007**
(21) Application number: 05816428.6
(22) Date of filing: 15.12.2005
(51) Int. Cl.: A61K 31/7088, A61K 48/00, A61P 1/02, A61P 19/00, A61P 19/02, A61P 19/08, A61P 19/10, A61P 29/00, A61P 35/00

(54) **AGENT FOR REGULATING BONE FORMATION**

(30) Priority: 16.12.2004 JP 2004365148; 12.08.2005 JP 2005234311
(71) Applicant: AnGes MG, Inc., Ibaraki-shi, Osaka 5670085 (JP)
(72) Inventor: SHIMIZU, Hideo, 6620077 (JP); NAKAGAMI, Hironori, 5670048 (JP); MORISHITA, Ryuichi, 5650851 (JP)
(74) Representative: Wichmann, Hendrik
(86) International application number: PCT/JP2005/023078
(87) International publication number: WO 2006/064886

(57) **Abstract**

The present invention provides preventive, ameliorating, and/or therapeutic agents for diseases caused by a disturbed balance between bone formation and bone resorption. The decoys of the present invention induce normal bone metabolism by inhibiting the differentiation-inducing factors of cells involved in bone metabolism. For example, bone resorption can be controlled by using a decoy of the present invention to inhibit NF-κB, a transcriptional regulatory factor that regulates osteoclast differentiation. This method uses a mechanism different from those of previous pharmaceutical agents; therefore, one can expect it to be effective for cases in which existing pharmaceutical agents were not effective.

SEQUENCE LISTING

<110>  ANGES MG, INC.

<120>  Agent for regulating bone formation

<130>  S64914PCEP

<140>  EP 05816428.6
<141>  2005-12-15

<150>  JP 2004-365148
<151>  2004-12-16

<150>  JP 2005-234311
<151>  2005-08-12

<160>  24

<170>  PatentIn version 3.3

<210>  1
<211>  10
<212>  DNA
<213>  Artificial Sequence

<220>
<223>  Description of Artificial Sequence: NF-κB consensus sequence

<400>  1
gggrhtyyhc                                                              10


<210>  2
<211>  20
<212>  DNA
<213>  Artificial Sequence

<220>
<223>  Description of Artificial Sequence: NF-κB decoy

<400>  2
ccttgaaggg atttccctcc                                                   20


<210>  3
<211>  9
<212>  DNA
<213>  Artificial Sequence

<220>
<223>  Description of Artificial Sequence: STAT-1 , STAT-3 consensus
       sequence


<220>
<221>  misc_feature
<222>  (4)..(6)
<223>  n is a, c, g, or t

<400>  3
ttcnnngaa                                                                9


<210>  4
<211>  9
<212>  DNA
<213>  Artificial Sequence

<220>
<223>  Description of Artificial Sequence: STAT-1, STAT-3 binding
      sequence

<400>  4
ttccgggaa                                                              9


<210>  5
<211>  10
<212>  DNA
<213>  Artificial Sequence

<220>
<223>  Description of Artificial Sequence: STAT-6 consensus sequence


<220>
<221>  misc_feature
<222>  (4)..(7)
<223>  n is a, c, g, or t

<400>  5
ttcnnnngaa                                                            10


<210>  6
<211>  10
<212>  DNA
<213>  Artificial Sequence

<220>
<223>  Description of Artificial Sequence: STAT-6 binding sequence

<400>  6
ttcccaagaa                                                            10


<210>  7
<211>  5
<212>  DNA
<213>  Artificial Sequence

<220>
<223>  Description of Artificial Sequence: Ets consensus sequence

<400>  7
mggaw                                                                  5


<210>  8
<211>  5
<212>  DNA
<213>  Artificial Sequence

<220>
<223>  Description of Artificial Sequence: Ets binding sequence

<400>  8
cggaa                                                                  5


<210>  9
<211>  7
<212>  DNA
<213>  Artificial Sequence

<220>
<223>  Description of Artificial Sequence: AP-1 consensus sequence

<400>  9
tgastma                                                                 7

<210>  10
<211>  7
<212>  DNA
<213>  Artificial Sequence

<220>
<223>  Description of Artificial Sequence: AP-1 binding sequence

<400>  10
tgagtca                                                                 7

<210>  11
<211>  8
<212>  DNA
<213>  Artificial Sequence

<220>
<223>  Description of Artificial Sequence: E2F consensus sequence

<400>  11
tttsscgs                                                                8

<210>  12
<211>  8
<212>  DNA
<213>  Artificial Sequence

<220>
<223>  Description of Artificial Sequence: E2F binding sequence

<400>  12
tttcccgc                                                                8

<210>  13
<211>  4
<212>  DNA
<213>  Artificial Sequence

<220>
<223>  Description of Artificial Sequence: Smad consensus sequence

<400>  13
gtct                                                                    4

<210>  14
<211>  8
<212>  DNA
<213>  Artificial Sequence

<220>
<223>  Description of Artificial Sequence: Smad binding sequence

<400>  14
gtctagac                                                                8

<210>  15
<211>  4
<212>  DNA
<213>  Artificial Sequence

<220>
<223>  Description of Artificial Sequence: Smad consensus sequence

<400>  15
caga                                                                    4


<210>  16
<211>  6
<212>  DNA
<213>  Artificial Sequence

<220>
<223>  Description of Artificial Sequence: Smad binding sequence

<400>  16
cagaca                                                                  6


<210>  17
<211>  4
<212>  DNA
<213>  Artificial Sequence

<220>
<223>  Description of Artificial Sequence: Smad consensus sequence

<400>  17
gccg                                                                    4


<210>  18
<211>  6
<212>  DNA
<213>  Artificial Sequence

<220>
<223>  Description of Artificial Sequence: Runx2/Cbfa1 consensus
       sequence

<400>  18
accaca                                                                  6


<210>  19
<211>  7
<212>  DNA
<213>  Artificial Sequence

<220>
<223>  Description of Artificial Sequence: Runx2/Cbfa1 binding sequence

<400>  19
aaccaca                                                                 7


<210>  20
<211>  20
<212>  DNA
<213>  Artificial Sequence

<220>
<223>  Description of Artificial Sequence: scrambled decoy

<400>  20
ttgccgtacc tgacttagcc                                                  20

<210>  21
<211>  20
<212>  DNA
<213>  Artificial Sequence

<220>
<223>  Description of Artificial Sequence: scrambled decoy

<400>  21
ggagggaaat cccttcaagg                                                    20


<210>  22
<211>  20
<212>  DNA
<213>  Artificial Sequence

<220>
<223>  Description of Artificial Sequence: scrambled decoy

<400>  22
ggctaagtca ggtacggcaa                                                    20


<210>  23
<211>  10
<212>  DNA
<213>  Artificial Sequence

<220>
<223>  Description of Artificial Sequence: NF-κB binding sequence

<400>  23
gggatttccc                                                               10


<210>  24
<211>  10
<212>  DNA
<213>  Artificial Sequence

<220>
<223>  Description of Artificial Sequence: NF-κB binding sequence

<400>  24
gggactttcc                                                               10

## Description

### Technical Field

The present invention relates to pharmaceuticals comprising nucleic acid pharmaceutical agents comprising sequences that bind to arbitrary transcriptional regulatory factors involved in bone formation, and methods of use thereof.

### Background Art

Bones are reconstructed through repeated formation by osteoblasts, and degradation and resorption by osteoclasts. Ordinarily, bone formation and bone resorption are in a state of balance, and a mutual response mechanism exists between osteoblasts and osteoclasts. However, when the balance between the two is disturbed, bone metabolism abnormalities develop, such as osteoporosis or bone destruction due to chronic rheumatoid arthritis. Such bone metabolism abnormalities are becoming one of the large problems of the current aging society.

For example, osteoporosis is a disease frequently found among middle-aged and older people, and particularly among women, and symptoms appearing with advanced age include pain in the back and hip, as well as stooping of the back and bending of the hip when the bones are crushed due to the body's weight.

At present, calcium agents (for example, calcium aspartate, calcium gluconate, and calcium lactate), vitamin D preparations (for example, Alfacalcidol and Calcitriol), female hormone preparations (Estriol and Ipriflavone), vitamin K2 preparations (Menatetrenone), and such are being used for osteoporosis, but each of them have side effects (for calcium agents and vitamin D preparations, these side effects include hypercalcemia, calcium stones, nephropathy, kidney stones, nausea, diarrhea, constipation, headache, insomnia, malaise, and memory decline; for female hormone preparations (estrogen), they include carcinogenesis, thrombophlebitis, and myocardial infarctions; for Ipriflavone, they include peptic ulcers, gastrointestinal bleeding, dizziness, staggering, rashes, and anemia; and for vitamin K2 preparations, they include gastric distress, nausea, diarrhea, rashes, and headaches). Therefore, there is still a desire for very safe and effective pharmaceutical agents.

Further, Paget's disease and the like are examples of diseases caused by a disturbed balance between osteoclasts and osteoblasts. Paget's disease is a chronic disease of the skeleton in which growth of the bone in the diseased parts becomes abnormal, and the bone enlarges and softens. In Paget's disease, both osteoclasts and osteoblasts become excessively active at certain sites in the bone, and the rate of metabolic turnover at these sites increases significantly. The excessively active sites enlarge, but since they are structurally abnormal, they become weaker than normal sites.

Furthermore, modulation of balance between osteoclasts and osteoblasts can be used to prevent, ameliorate, or treat bone tumors, traumatic chondral defects, osteochondritis dissecans, osteoarthritis, rheumatoid arthritis, bone fractures, dislocations, periodontal diseases, and dental caries, and to ameliorate or treat conditions during or after orthodontic and artificial dental implant treatments.

For example, in bone tumors, tumors originating from various tissues eventually cause bone resorption (bone destruction), and induce bone fracture, bone pain, and hypercalcemia. There are two types of bone resorption: local bone resorption in which tumor cells migrate directly to the bone, activate osteoclasts at that site, and lead to bone destruction; and systemic bone resorption in which bone resorption-promoting factors such as parathyroid hormone-related protein (PTH-rP) produced from tumors activate osteoclasts and lead to bone destruction. In either case, the modulation of osteoclasts may prevent, ameliorate, or treat the disease.

In rheumatoid arthritis, inflammation is said to be followed by the induction of inflammatory cytokines such as IL-1, TNF-α, and IL-6, leading to osteoclast formation and bone resorption. Therefore, suppression of bone resorption by modulating osteoclasts may prevent, ameliorate, or treat diseases.

Maintenance of such balance by osteoclasts and osteoblasts is not limited to bones, but also takes place in teeth.

For example, in periodontal diseases, osteoclasts are activated by the toxins of anaerobic eubacteria, and the bone is destroyed; however, in this case as well, inactivation of osteoclasts may lead to prevention, amelioration, or treatment.

Orthodontics utilizes the phenomenon by which a force applied to a tooth from a certain direction causes osteoclasts that appear in the part of the bone where the force was applied to destroy and resorb the bone, the tooth moves to the region resorbed by the osteoclasts, and osteoblasts that appear at the region after displacement then regenerate the bone. In these cases also, it is thought that treatment may be ameliorated by modulating the balance between osteoclasts and osteoblasts.

In this context, recent attempts have been made to develop novel bone formation-modulating agents, or more specifically, to develop pharmaceutical agents with the activity of modulating the differentiation of osteoclasts and osteoblasts.

Patent Document 1 reports that lactacystin, peptidyl aldehyde, pentoxyfilline (PTX), and such can regulate bone formation and hair growth by inhibiting proteasomes or NF-κB.

However, in Example 4 of Patent Document 1, the inventors reveal that in an *in vitro* calvaria assay, those compounds that inhibit NF-κB but do not inhibit proteasome activity do not enhance bone formation to the pharmacologically active levels demonstrated by proteasome inhibitors.

The invention of Patent Document 2 discloses a method for treating diseases characterized by undesirable bone resorption using an NMDA-receptor antagonist. It also discloses that antisense mRNAs with the ability to inhibit translation ofNMDA receptor mRNAs in osteoclasts may also be used for such treatment.

However, although the inventors show Examples relating to known NMDA-receptor antagonist MK-801 (Merck) and phencyclidine (PCP), they do not give specific examples of antisense mRNAs which are nucleic acid pharmaceuticals.

The invention of Patent Document 3 relates to methods for enhancing bone formation by administering an effective dose of one or more oligomer complexes selected from among RANKL (OPGL), RANKL fusion proteins, analogs, derivatives, or mimics, or osteogenic compounds.

The Examples of Patent Document 3 disclose that administration of GST-RANKL stimulates the proliferation of osteoblasts; however, this method calls for administration of a protein, which differs from the nucleic acid pharmaceuticals of the present invention. Furthermore, although RANKL has been identified as a potent inducer of bone resorption and a positive regulatory factor for osteoclast development, the focus in Patent Document 3 is on the stimulation of osteoblasts by RANKL, and thus it differs from the present invention.
[Patent Document 1] WO 00/02548 pamphlet.
[Patent Document 2] Japanese Patent Kohyo Publication No. (JP-A) 2001-513757 (unexamined Japanese national phase publication corresponding to a non-Japanese international publication).
[Patent Document 3] JP-A 2004-526748.
[Non-Patent Document 1] Garrett I. R. *et al.,* J. Clin. Invest., 2003, 111(11); 1771-1782.

### Disclosure of the Invention

### Problems to be Solved by the Invention

A problem to be solved by the present invention is the development of preventive agents, ameliorating agents, and/or therapeutic agents for diseases caused by a disturbed balance between osteoclasts and osteoblasts.

### Means to Solve the Problems

The present inventors developed pharmaceutical agents with the activity of modulating the differentiation of osteoclasts and osteoblasts.

When bone resorption exceeds bone formation, bone mass and bone density become insufficient, but in such cases, the inhibition of bone resorption can prevent, ameliorate, and/or treat diseases.

The differentiation of osteoclasts, which play an important role in bone resorption, is regulated by various hormones and cytokines, such as IL-1 and TNF-α. Recently, it has also been revealed that osteoclast differentiation requires RANK (Receptor Activator of NF-κB)-RANKL (Receptor Activator of NF-κB Ligand) signal transduction through cell-cell contact.

As the result of dedicated research, the present inventors discovered that osteocyte differentiation can be modulated by inhibiting transcriptional regulatory factors using decoys.

More specifically, the present inventors used decoys to inhibit NF-κB, which is heavily involved in osteoclast differentiation, and discovered that these suppress osteoclast differentiation.

Therefore, the present invention provides bone formation modulating agents comprising nucleic acids comprising bone formation-related transcriptional regulatory factor binding sequence as active ingredients. Specifically, the present invention provides nucleic acid pharmaceutical agents (decoys) that inhibit transcriptional regulatory factors involved in osteocyte differentiation to correct a disturbed balance between bone formation and bone resorption.

In summary, the present invention relates to:
(1) a bone formation modulating agent, in which a nucleic acid comprising a bone formation-related transcriptional regulatory factor binding sequence is an active ingredient;
(2) the bone formation modulating agent of (1), wherein the nucleic acid is selected from a group consisting of a decoy, an antisense, a ribozyme, an aptamer, and an siRNA;
(3) the bone formation modulating agent of (1), wherein the nucleic acid is a decoy comprising a DNA or an RNA;
(4) the bone formation modulating agent of (2) or (3), wherein the decoy comprises a DNA oligonucleotide;
(5) the bone formation modulating agent of any one of (2) to (4), wherein the decoy is a decoy of NF-κB, STAT-1, STAT-3, STAT-6, Ets, AP-1, E2F, Smad1 Smad2, Smad3, Smad4, Smad5, Smad6, Smad7, Smad8, or Runx2/Cbfa1;
(6) the bone formation modulating agent of (5), wherein the decoy is a decoy of NF-κB;
(7) the bone formation modulating agent of any one of (2) to (6), wherein the decoy comprises the NF-κB binding sequence GGGRHTYYHC (wherein R means A or G, Y means C or T, and H means A, C, or T);
(8) the bone formation modulating agent of (7), wherein the decoy comprises the NF-κB binding sequence GGGATTTCCC (SEQ ID NO: 23) or GGGACTTTCC (SEQ ID NO: 24);
(9) the bone formation modulating agent of any one of (2) to (8), wherein the decoy is a decoy represented by SEQ ID NO: 2;
(10) the bone formation modulating agent of any one of (2) to (5), wherein the decoy comprises the STAT-1 or STAT-3 binding sequence TTCNNNGAA (wherein N means A, Q T, or C);
(11) the bone formation modulating agent of (10), wherein the decoy comprises the STAT-1 or STAT-3 binding sequence TTCCGGGAA;
(12) the bone formation modulating agent of any one of (2) to (5), wherein the decoy comprises the STAT-6 binding sequence TTCNNNNGAA (wherein N means A, G, T, or C);
(13) the bone formation modulating agent of (12), wherein the decoy comprises the STAT-6 binding sequence TTCCCAAGAA;
(14) the bone formation modulating agent of any one of (2) to (5), wherein the decoy comprises the Ets binding sequence MGGAW (wherein M means A or C, and W means A or T);
(15) the bone formation modulating agent of (14), wherein the decoy comprises the Ets binding sequence CGCTAA;
(16) the bone formation modulating agent of any one of (2) to (5), wherein the decoy comprises the AP-1 binding sequence TGASTMA (wherein S means G or C, and M means A or C);
(17) the bone formation modulating agent of (16), wherein the decoy comprises the AP-1 binding sequence TGAGTCA;
(18) the bone formation modulating agent of any one of (2) to (5), wherein the decoy comprises the E2F binding sequence TTTSSCGS (wherein S means G or C);
(19) the bone formation modulating agent of (18), wherein the decoy comprises the E2F binding sequence TTTCCCGC;
(20) the bone formation modulating agent of any one of (2) to (5), wherein the decoy comprises the Smad1, Smad2, Smad3, Smad4, Smad5, Smad6, Smad7, or Smad8 binding sequence GTCT, CAGA, or GCCG;
(21) the bone formation modulating agent of (20), wherein the decoy comprises the Smad1, Smad2, Smad3, Smad4, Smad5, Smad6, Smad7, or Smad8 binding sequence GTCTAGAC or CAGACA;
(22) the bone formation modulating agent of any one of (2) to (5), wherein the decoy comprises the Runx2/Cbfa1 binding sequence ACCACA;
(23) the bone formation modulating agent of (22), wherein the decoy comprises the Runx2/Cbfa1 binding sequence AACCACA;
(24) the bone formation modulating agent of any one of (2) to (23), wherein the decoy comprises a double-stranded DNA;
(25) a preventive agent, ameliorating agent, or therapeutic agent for a disease caused by a disturbed balance between osteoclasts and osteoblasts, wherein the agent comprises the bone formation modulating agent of any one of (1) to (24);
(26) a preventive agent, ameliorating agent, or therapeutic agent, wherein the disease of (25) is any one selected from osteoporosis, Paget's disease, bone tumor, traumatic chondral defect, osteochondritis dissecans, osteoarthritis, rheumatoid arthritis, bone fracture, dislocation, periodontal disease, and dental caries;
(27) an ameliorating agent for an orthodontic or artificial dental implant treatment, which comprises the bone formation modulating agent of any one of (1) to (24);
(28) a method for modulating bone formation that uses a nucleic acid comprising a bone formation-related transcriptional regulatory factor binding sequence;
(29) the method of (28), wherein the nucleic acid is selected from a group consisting of a decoy, an antisense, a ribozyme, an aptamer, and an siRNA;
(30) the method of (28), wherein the nucleic acid is a decoy comprising a DNA or an RNA;
(31) the method of (29) or (30), wherein the decoy comprises a DNA oligonucleotide;
(32) the method of any one of (29) to (31), wherein the decoy is a decoy of NF-κB, STAT-1, STAT-3, STAT-6, Ets, AP-1, E2F, Smad1, Smad2, Smad3, Smad4, Smad5, Smad6, Smad7, Smad8, or Runx2/Cbfa1;
(33) the method of (32), wherein the decoy is a decoy of NF-κB;
(34) the method of any one of (29) to (33), wherein the decoy comprises the NF-κB binding sequence GGGRHTYYHC (wherein R means A or G, Y means C or T, and H means A, C, or T);
(35) the method of (34), wherein the decoy comprises the NF-κB binding sequence GGGATTTCCC (SEQ ID NO: 23) or GGGACTTTCC (SEQ ID NO: 24);
(36) the method of any one of (29) to (35), wherein the decoy is a decoy represented by SEQ ID NO: 2;
(37) the method of any one of (29) to (32), wherein the decoy comprises the STAT-1 or STAT-3 binding sequence TTCNNNGAA (wherein N means A, G, T, or C);
(38) the method of (37), wherein the decoy comprises the STAT-1 or STAT-3 binding sequence TTCCGGGAA;
(39) the method of any one of (29) to (32), wherein the decoy comprises the STAT-6 binding sequence TTCNNNNGAA (wherein N means A, G, T, or C);
(40) the method of (39), wherein the decoy comprises the STAT-6 binding sequence TTCCCAAGAA;
(41) the method of any one of (29) to (32), wherein the decoy comprises the Ets binding sequence MGGAW (wherein M means A or C, and W means A or T);
(42) the method of (41), wherein the decoy comprises the Ets binding sequence CGGAA;
(43) the method of any one of (29) to (32), wherein the decoy comprises the AP-1 binding sequence TGASTMA (wherein S means G or C, and M means A or C);
(44) the method of (43), wherein the decoy comprises the AP-1 binding sequence TGAGTCA;
(45) the method of any one of (29) to (32), wherein the decoy comprises the E2F binding sequence TTTSSCGS (wherein S means G or C);
(46) the method of (45), wherein the decoy comprises the E2F binding sequence TTTCCCGC;
(47) the method of any one of (29) to (32), wherein the decoy comprises the Smad1, Smad2, Smad3, Smad4, Smad5, Smad6, Smad7, or Smad8 binding sequence GTCT, CAGA, or GCCG;
(48) the method of (47), wherein the decoy comprises the Smad1, Smad2, Smad3, Smad4, Smad5, Smad6, Smad7, or Smad8 binding sequence GTCTAGAC or CAGACA;
(49) the method of any one of (29) to (32), wherein the decoy comprises the Runx2/Cbfa1 binding sequence ACCACA;
(50) the method of (49), wherein the decoy comprises the Runx2/Cbfal binding sequence AACCACA;
(51) the method of any one of (29) to (50), wherein the decoy comprises a double-stranded DNA;
(52) the method of any one of (28) to (51) for preventing, ameliorating, or treating a disease caused by a disturbed balance between osteoclasts and osteoblasts;
(53) the method of (52), wherein the disease caused by the disturbed balance between osteoclasts and osteoblasts is selected from the group consisting of osteoporosis, Paget's disease, bone tumor, traumatic chondral defect, osteochondritis dissecans, osteoarthritis, rheumatoid arthritis, bone fracture, dislocation, periodontal disease, and dental caries;
(54) the method of any one of (28) to (51) for ameliorating an orthodontic or artificial dental implant treatment;
(55) a use of a nucleic acid comprising a bone formation-related transcriptional regulatory factor binding sequence for producing a pharmaceutical agent for modulating bone formation;
(56) the use of (55), wherein the nucleic acid is selected from a group consisting of a decoy, an antisense, a ribozyme, an aptamer, and an siRNA;
(57) the use of (55), wherein the nucleic acid is a decoy comprising a DNA or an RNA;
(58) the use of (56) or (57), wherein the decoy comprises a DNA oligonucleotide;
(59) the use of any one of (56) to (58), wherein the decoy is a decoy of NF-κB, STAT-1, STAT-3, STAT-6, Ets, AP-1, E2F, Smad1, Smad2, Smad3, Smad4, Smad5, Smad6, Smad7, Smad8, or Runx2/Cbfa1;
(60) the use of (59), wherein the decoy is a decoy of NF-κB;
(61) the use of any one of (56) to (60), wherein the decoy comprises the NF-κB binding sequence GGGRHTYYHC (wherein R means A or G, Y means C or T, and H means A, C, or T);
(62) the use of (61), wherein the decoy comprises the NF-κB binding sequence GGGATITCCC (SEQ ID NO: 23) or GGGACTTTCC (SEQ ID NO: 24);
(63) the use of any one of (56) to (62), wherein the decoy is a decoy represented by SEQ ID NO: 2;
(64) the use of any one of (56) to (59), wherein the decoy comprises the STAT-1 or STAT-3 binding sequence TTCNNNGAA (wherein N means A, G, T, or C);
(65) the use of (64), wherein the decoy comprises the STAT-1 or STAT-3 binding sequence TTCCGGGAA;
(66) the use of any one of (56) to (59), wherein the decoy comprises the STAT-6 binding sequence TRCNNNNGAA (wherein N means A, G, T, or C);
(67) the use of (66), wherein the decoy comprises the STAT-6 binding sequence TTCCCAAGAA;
(68) the use of any one of (56) to (59), wherein the decoy comprises the Ets binding sequence MGGAW (wherein M means A or C, and W means A or T);
(69) the use of (68), wherein the decoy comprises the Ets binding sequence CGGAA;
(70) the use of any one of (56) to (59), wherein the decoy comprises the AP-1 binding sequence TGASTMA (wherein S means G or C, and M means A or C);
(71) the use of (70), wherein the decoy comprises the AP-1 binding sequence TGAGTCA;
(72) the use of any one of (56) to (59), wherein the decoy comprises the E2F binding sequence TTTSSCGS (wherein S means G or C);
(73) the use of (72), wherein the decoy comprises the E2F binding sequence TTTCCCGC;
(74) the use of any one of (56) to (59), wherein the decoy comprises the Smad1, Smad2, Smad3, Smad4, Smad5, Smad6, Smad7, or Smad8 binding sequence GTCT, CAGA, or GCCG;
(75) the use of (74), wherein the decoy comprises the Smad1, Smad2, Smad3, Smad4, Smad5, Smad6, Smad7, or Smad8 binding sequence GTCTAGAC or CAGACA;
(76) the use of any one of (56) to (59), wherein the decoy comprises the Runx2/Cbfa1 binding sequence ACCACA;
(77) the use of (76), wherein the decoy comprises the Runx2/Cbfa1 binding sequence AACCACA;
(78) the use of any one of (56) to (77), wherein the decoy comprises a double-stranded DNA;
(79) the use of any one of (55) to (78), wherein the bone formation modulating agent prevents, ameliorates, or treats a disease caused by a disturbed balance between osteoclasts and osteoblasts;
(80) the use of (79), wherein the disease caused by a disturbed balance between osteoclasts and osteoblasts is selected from the group consisting of osteoporosis, Paget's disease, bone tumor, traumatic chondral defect, osteochondritis dissecans, osteoarthritis, rheumatoid arthritis, bone fracture, dislocation, periodontal disease, and dental caries; and
(81) the use of any one of (55) to (78), wherein the bone formation modulating agent ameliorates an orthodontic or artificial dental implant treatment.

### Effects of the Invention

The present invention provided decoys that are nucleic acid pharmaceuticals that inhibit transcriptional regulatory factors involved in bone formation. Furthermore, such decoys can be used for diseases caused by a disturbed balance between osteoclasts and osteoblasts, such as osteoporosis and Paget's disease. In addition, they can be used as preventive, ameliorating, or therapeutic agents for bone tumors, traumatic chondral defects, osteochondritis dissecans, osteoarthritis, rheumatoid arthritis, bone fractures, dislocations, periodontal diseases, and dental caries, as well as ameliorating agents for orthodontic and artificial dental implant treatments.

### Brief Description of the Drawings

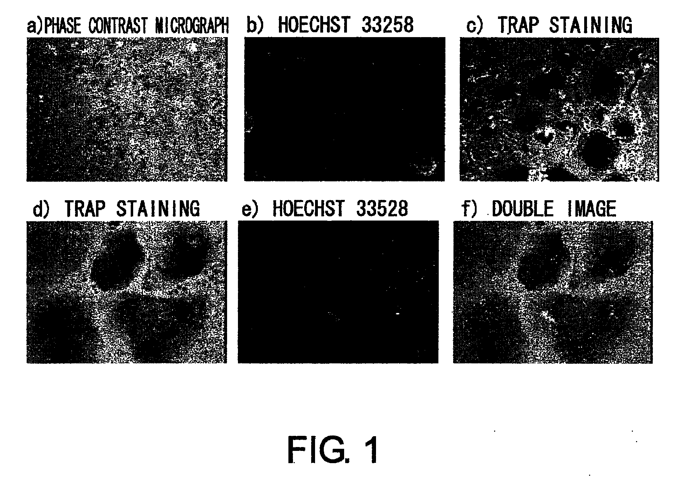

Fig. 1 shows that multinucleated cells are formed during differentiation from bone marrow cells: It shows (a) a phase contrast micrograph (x 40), (b) a Hoechst 33258 staining image (x 40), and (c) a TRAP staining image (x 40) of bone marrow cells induced to differentiate by treatment for seven days with vitamin D3; as well as (d) a TRAP staining image (x 100), (e) a Hoechst 33258 staining image (x 100), and (f) a double image of TRAP and Hoechst 33258 staining (x 100) of NF-κB decoy-treated cells.

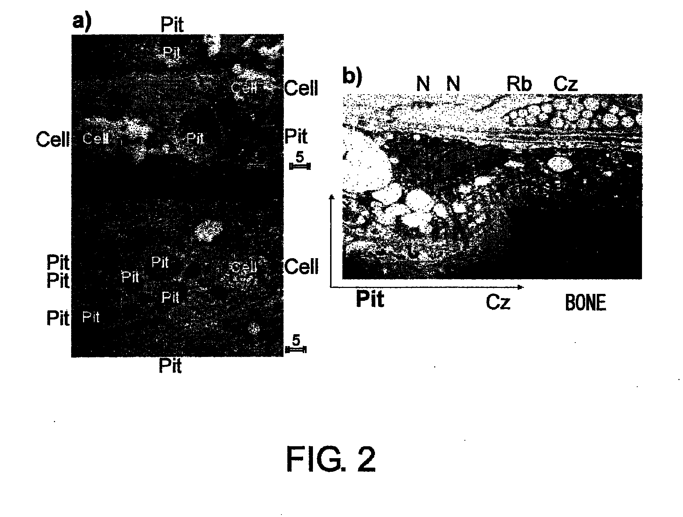

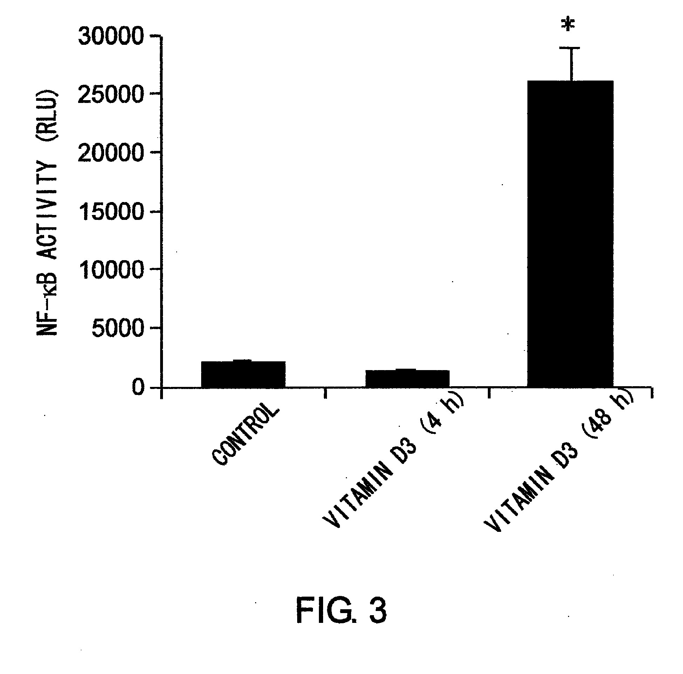

Fig. 2 shows (a) a scanning electron micrograph of bone marrow cells induced to differentiate by treatment for seven days with vitamin D3 ('Cell' refers to osteoclasts, and 'Pit' refers to resorption pits; the bar corresponds to 5 µm; magnification is x 1000), and (b) a transmission electron micrograph of a resorption pit section (N, Cz, and Rb refer to the nucleus, clear zone, and ruffled border, respectively; the arrows show the size of a single resorption pit; magnification is x 1,000,000). Multinucleated cells tightly covering the bone surface with a clear zone (Cz) absorb the bone minerals through the ruffled borders (Rb). Osteoclasts leave excavated pits on the surface and move on the bone surface.
Fig. 3 shows the effects of vitamin D3 (1 x 10⁻⁸ M) on luciferase activity regulated by NF-κB binding sequences in bone marrow cells. (* P < 0.01, N = 6)

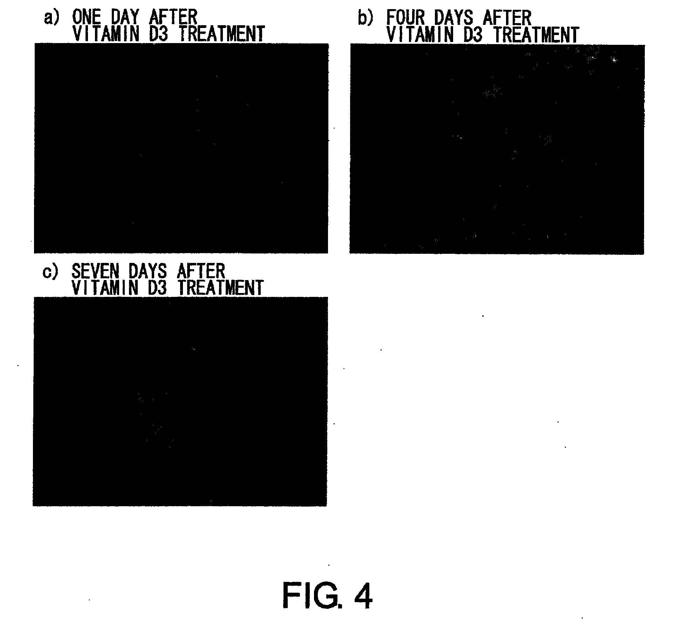

Fig. 4 shows fluorescence micrographs of cells transfected with FITC-labeled NF-κB decoy one, four and seven days after vitamin D3 treatment (a, b: x 40; c: x 100). The NF-κB decoy was incorporated into osteoclasts at all stages of differentiation.

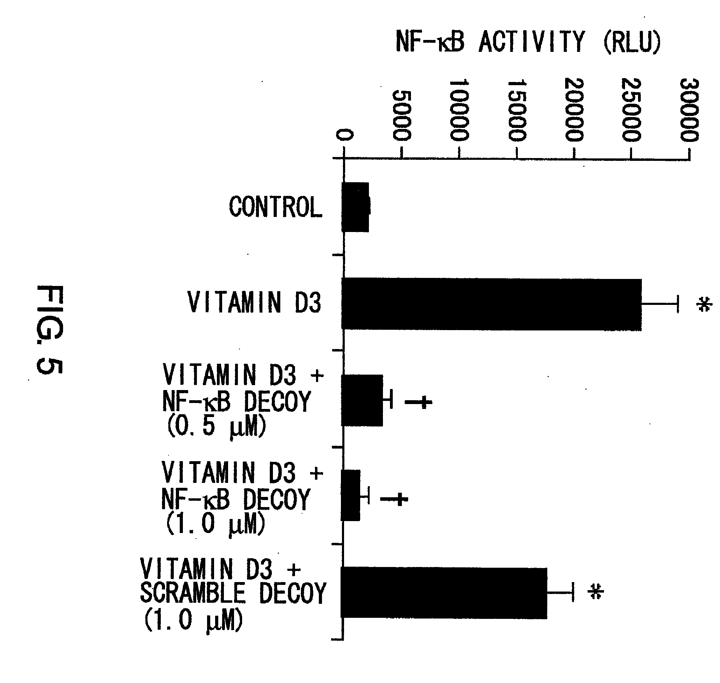

Fig. 5 shows the luciferase activity of cells transfected with FITC-labeled NF-κB decoy four days after vitamin D3 treatment. (* P < 0.01 compared to the control; † P < 0.01 compared to vitamin D3;N= 6)

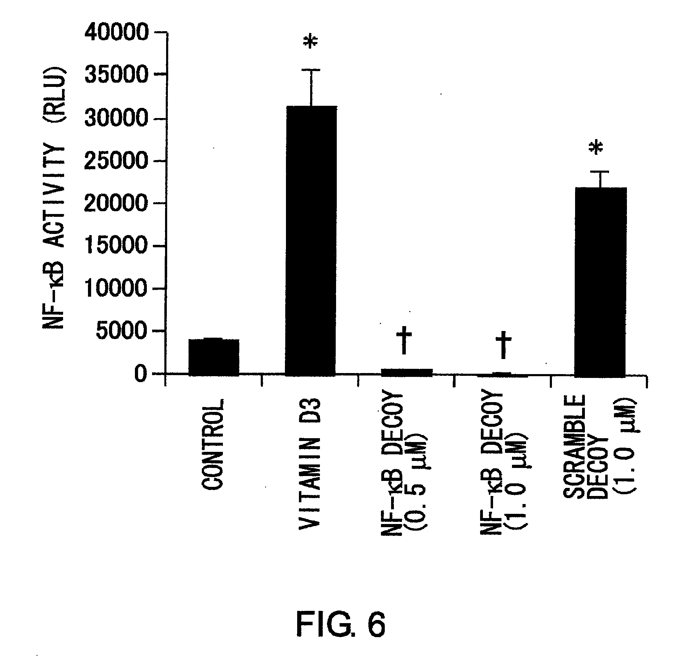

Fig. 6 shows the luciferase activity of cells transfected with FITC-labeled NF-κB decoy seven days after vitamin D3 treatment. (* P < 0.01 compared to the control; † P < 0.01 compared to vitamin D3; N = 6)

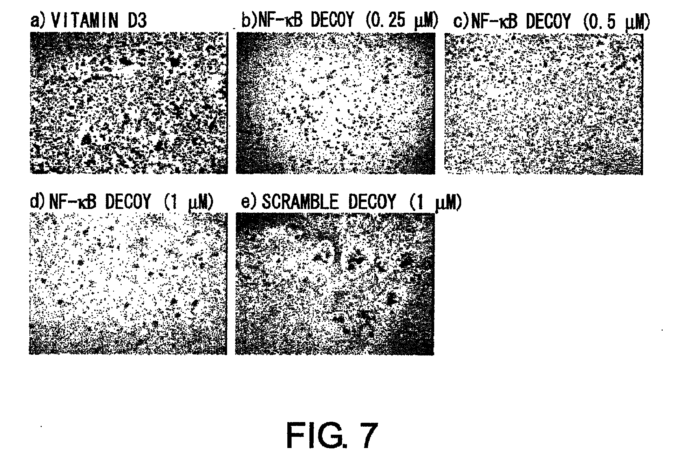

Fig. 7 shows micrographs (x 40) of TRAP-positive cells seven days after vitamin D3 treatment of cells transfected with FITC-labeled NF-κB decoy or scramble decoy. a) Vitamin D3: treated with vitamin D3 (1 x 10⁻⁸ M) alone; b) NF-κB decoy (0.25 µM): treated with vitamin D3 and NF-κB decoy (0.25 µM); c) NF-κB decoy (0.5 µM): treated with vitamin D3 and NF-κB decoy (0.5 µM); d) NF-κB decoy (1 µM): treated with vitamin D3 and NF-κB decoy (1 µM); and e) Scramble decoy (1 µM): treated with vitamin D3 and scramble decoy (1 µM). (* P < 0.01 compared to treatment with vitamin D3 alone; N = 6)

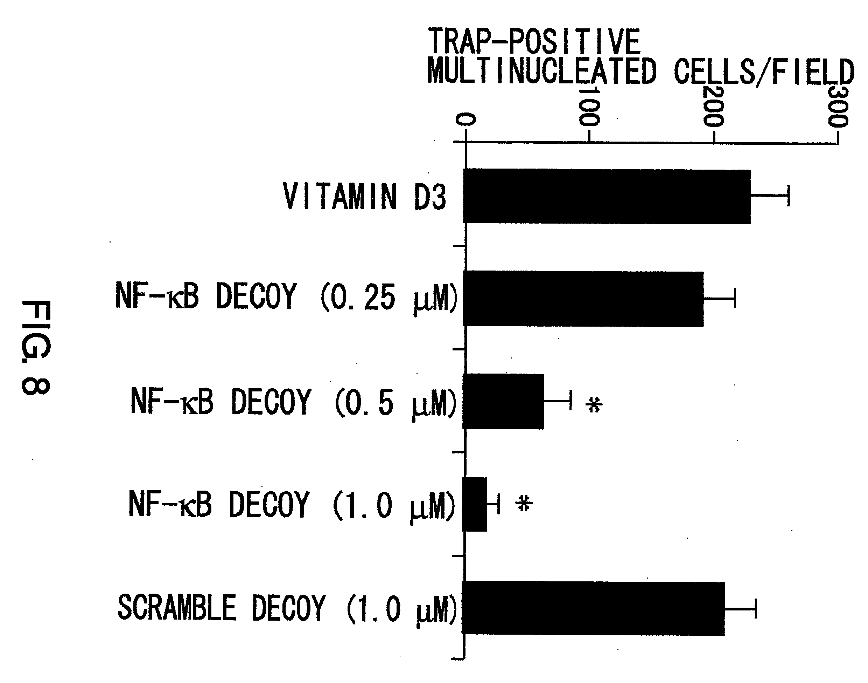

Fig. 8 shows the effects of NF-κB decoy on TRAP-positive cells. (N = 6)

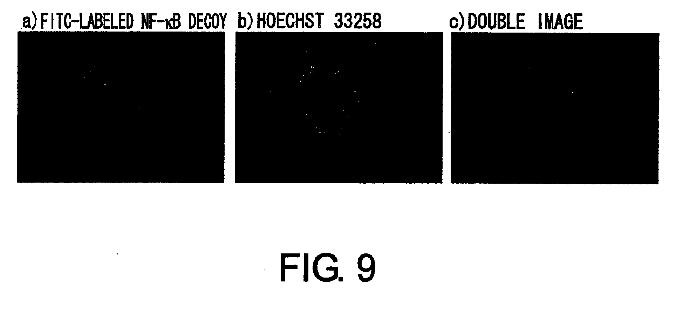

Fig. 9 shows (a) a fluorescence micrograph of osteoclasts transfected with FITC-labeled NF-κB decoy, (b) a Hoechst 33258 staining image, and (c) a double image of FITC and Hoechst 33258 staining (x 100).

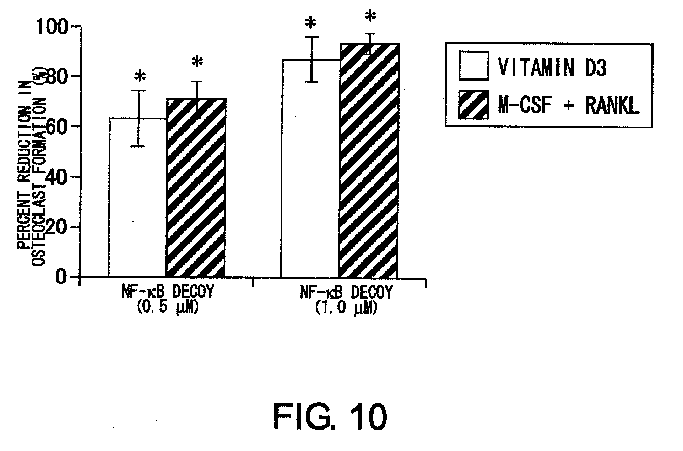

Fig. 10 shows the influence of NF-κB decoy on cells that differentiated into osteoclasts due to vitamin D3, or M-CSF and RANKL. The percentage reduction in osteoclast formation (%) is indicated as the proportion of the number of TRAP-positive cells that decreased as compared to the scramble decoy group. Vitamin D3: stimulation with vitamin D3; M-CSF + RANKL: stimulation with RANKL and M-CSF (* P < 0.01; N = 6)

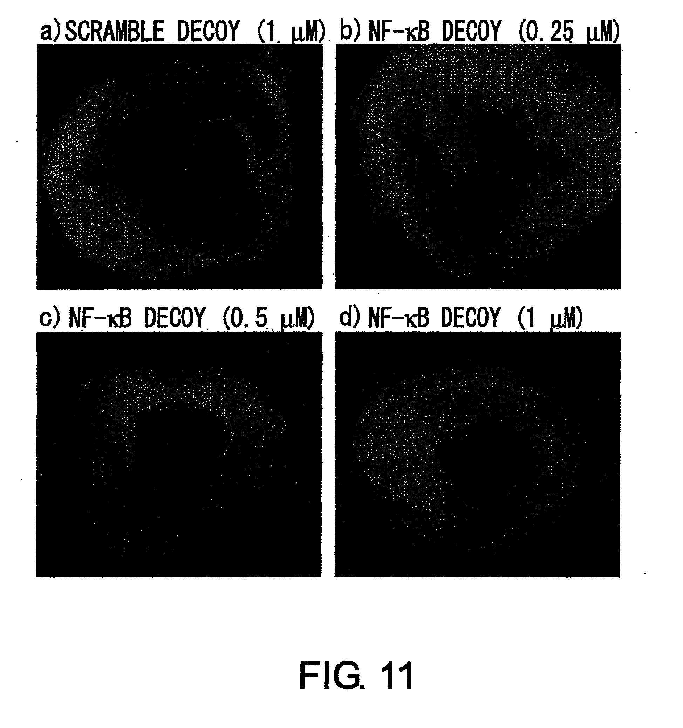

Fig. 11 shows micrographs (x 5) of dentine sections, to which NF-κB decoy or scramble decoy was added, at three days after vitamin D3 treatment. Scramble decoy (1 µM): treated with vitamin D3 (1 x 10⁻⁸ M) and scramble decoy (1 µM); NF-κB decoy (0.25 µM, 0.5 µM, and 1 µM): treated with vitamin D3 (1 x 10⁻⁸ M) and NF-κB decoy (0.25 µM, 0.5 µM, or 1 µM).

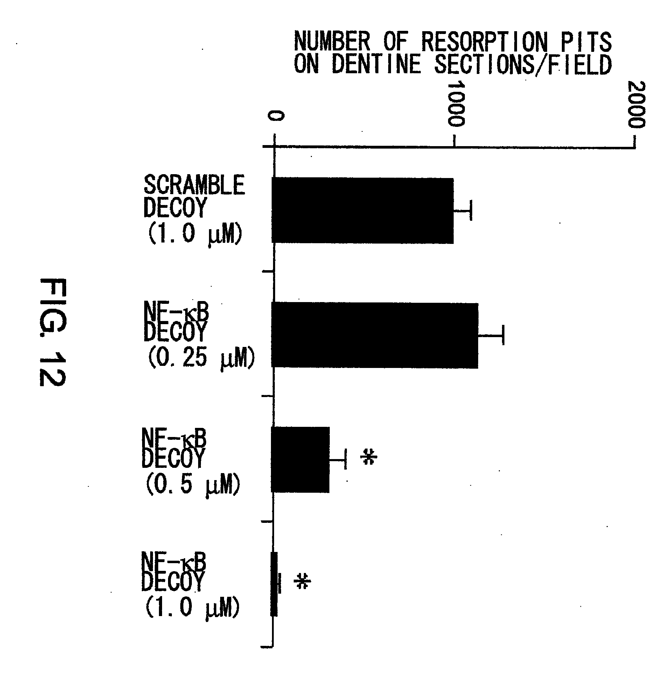

Fig. 12 shows the number of resorption pits on dentine sections to which NF-κB decoy or scramble decoy was added, at three days after vitamin D3 treatment. Quantitative analysis of Fig. 11. (* P < 0.01 compared to the scrambled decoy group)

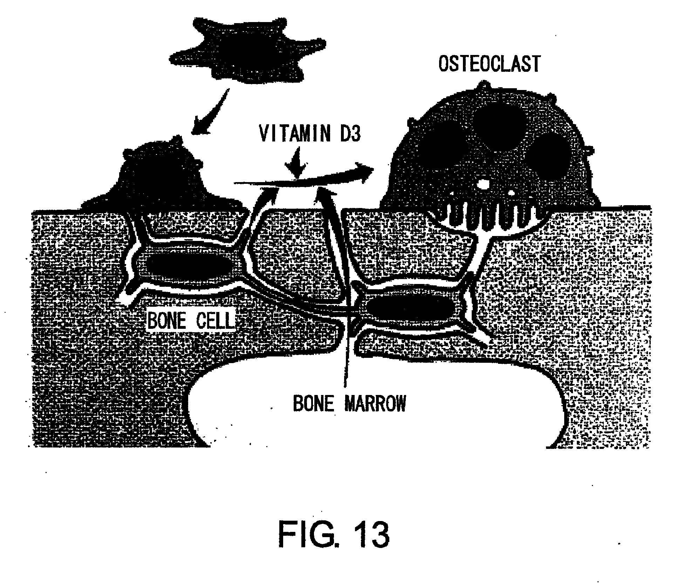

Fig. 13 shows a schematic diagram of osteoclast activation and resorption pit formation.

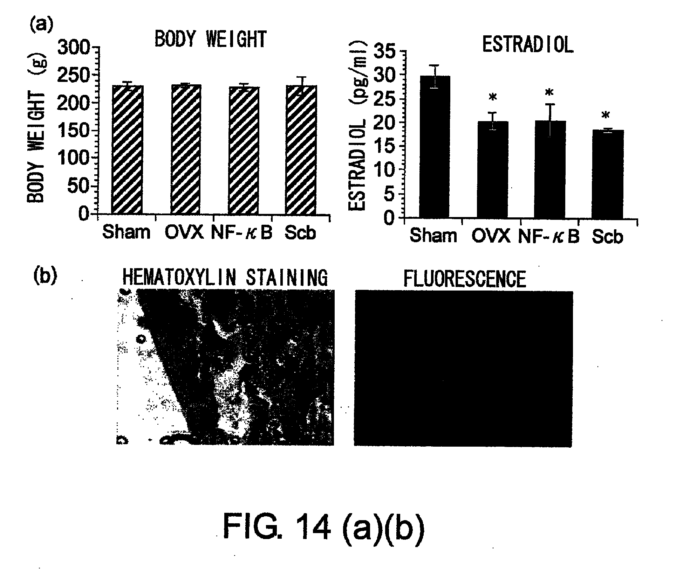

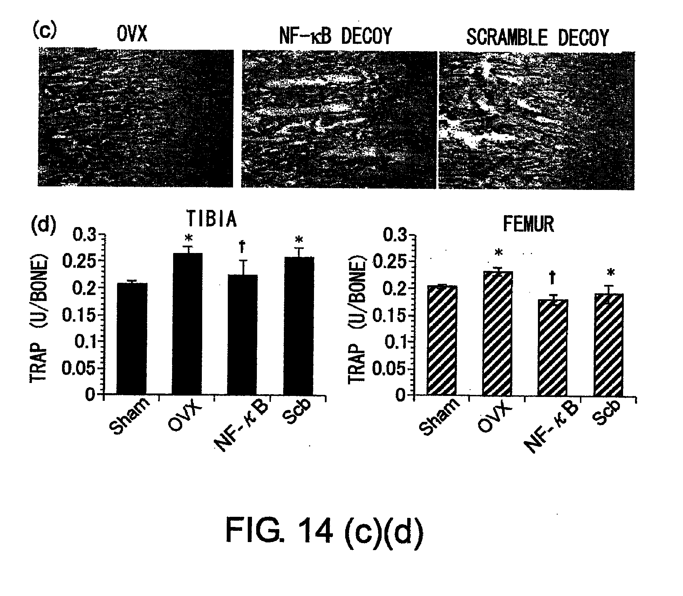

Fig. 14(a) and (b) show the effects of NF-κB decoy in the ovariectomy-induced osteoporosis model. (a) shows the body weight and serum estradiol levels. (b) The left panel shows a micrograph after hematoxylin staining and the right panel shows a fluorescence micrograph of the cryosections of proximal tibia after FITC-labeled NF-κB decoy treatment (30 µg/kg/h) (x 40). Sham = sham operation; OVX = bilateral ovariectomy; NF-κB = bilateral ovariectomy and treatment with NF-κB decoy (30 µg/kg/h); Scb = scramble decoy (30 µg/kg/h) = bilateral ovariectomy and treatment with scramble decoy (30 µg/kg/h). (* P < 0.05 compared to Sham; † P < 0.05 compared to Scb; N = 8)
Fig. 14(c) and (d) show the effects of NF-κB decoy in the ovariectomy-induced osteoporosis model. (c) TRAP staining images of the distal femur at 14 days after treatment (x 100); and (d) TRAP activities in the proximal tibia and distal femur in four groups at 14 days after treatment. Sham = sham operation; OVX = bilateral ovariectomy; NF-κB = bilateral ovariectomy and treatment with NF-κB decoy (30 µg/kg/h); Scb = scramble decoy (30 µg/kg/h) = bilateral ovariectomy and treatment with scramble decoy (30 µg/kg/h). (* P < 0.05 compared to Sham. † P < 0.05 compared to Scb; N = 8)

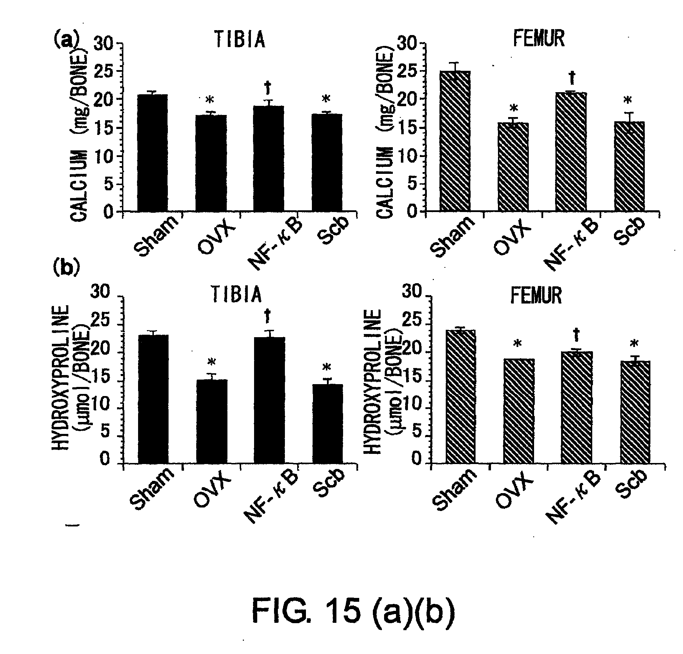

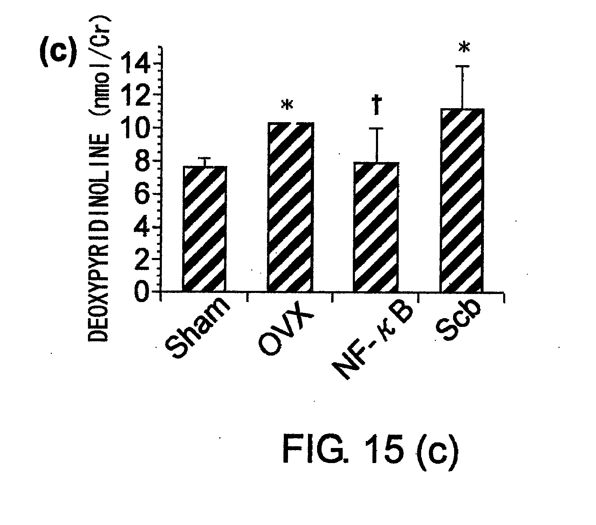

Fig. 15(a) and (b) show the effects of NF-κB decoy on (a) calcium concentrations in proximal tibia and distal femur and (b) hydroxyproline concentrations in proximal tibia and distal femur in four groups of ovariectomy-induced osteoporosis models at 14 days after treatment. Sham = sham operation; OVX = bilateral ovariectomy; NF-κB = bilateral ovariectomy and treatment with NF-κB decoy (30 µg/kg/h); Scb = scramble decoy (30 µg/kg/h) = bilateral ovariectomy and treatment with scramble decoy (30 µg/kg/h). (* P < 0.05 compared to Sham; † P < 0.05 compared to Scb; N = 8)
Fig. 15(c) shows the effects of NF-κB decoy on (c) urinary deoxypyridinoline (which is regulated by the urinary creatine concentration) in four groups of ovariectomy-induced osteoporosis models at 14 days after treatment. Sham = sham operation; OVX = bilateral ovariectomy; NF-κB = bilateral ovariectomy and treatment with NF-κB decoy (30 µg/kg/h); Scb = scramble decoy (30 µg/kg/h) = bilateral ovariectomy and treatment with scramble decoy (30 µg/kg/h). (* P < 0.05 compared to Sham; † P < 0.05 compared to Scb; N = 8)

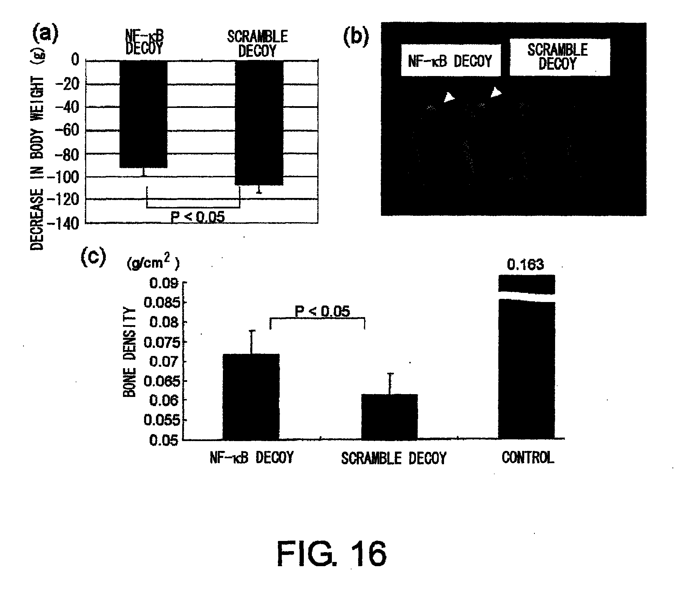

Fig. 16 (a) is a graph showing the effect of NF-κB decoy ODN on the reduction in body weight after terminating L-ascorbate administration. NF-κB decoy ODN treatment suppressed the reduction in body weight. (b) shows a photograph of the femurs of scramble decoy ODN-treated rats and NF-κB decoy ODN-treated rats. The femoral head (part indicated with an arrow) was not destroyed and was preserved only in the NF-κB decoy ODN-treated group. (c) is a graph showing the effect of NF-κB decoy ODN on bone density as assessed by dual-energy X-ray absorptiometry analysis at 28 days after treatment in the osteogenic disorder Shionogi rat models. NF-κB decoy = osteogenic disorder Shionogi rats were fed on a vitamin C-deficient diet and administered 30 µg/kg/h of NF-κB decoy ODN; Scramble decoy = osteogenic disorder Shionogi rats were fed on a vitamin C-deficient diet and administered 30 µg/kg/h of scramble decoy ODN; Control = age-matched normal Wistar rats were fed on a vitamin C-deficient diet. * P < 0.05 compared to scramble decoy; N = 6 per group.

### Best Mode for Carrying Out the Invention

The bone formation modulating agents of the present invention are nucleic acid pharmaceutical agents that comprise nucleic acids comprising bone formation-related transcriptional regulatory factor binding sequence as active ingredients. The bone formation modulating agents of the present invention can modulate the process of bone formation (for example, the balance between osteoclasts and osteoblasts) by acting on bone formation-related transcriptional regulatory factors.

Various factors relating to bone formation are included in the "bone formation-related transcriptional regulatory factors" described in the present description. Specific examples of bone formation-related transcriptional regulatory factors include NF-κB, STAT-1, STAT-3, STAT-6, Ets, AP-1, E2F, Smad1, Smad2, Smad3, Smad4, Smad5, Smad6, Smad7, Smad8, and/or Runx2/Cbfa1, but are not limited thereto. In the present description, a "bone formation-related transcriptional regulatory factor binding sequence" means a sequence that can bind to a transcriptional regulatory factor involved in bone formation, such as those transcriptional regulatory factors mentioned above.

The nucleic acid pharmaceutical agents used in the present invention are ordinarily decoys, antisenses, ribozymes, aptarners, siRNAs, or such, but are preferably decoys. In the present description, decoys, decoy oligonucleotides, and decoy ODNs are used synonymously

Examples of decoys that may be used include decoys of NF-κB, STAT-1, STAT-3, STAT-6, Ets, AP-1, E2F, Smad1, Smad2, Smad3, Smad4, Smad5, Smad6, Smad7, Smad8, and Runx2/Cbfa1, but NF-κB decoys are preferred. In one embodiment, NF-κB decoys comprise an NF-κB-binding sequence. An NF-κB-binding sequence can be selected from among the consensus sequences of NF-κB binding sequence, GGGRHTYYHC (R(A,G); Y(C, T); and H(A, C, T)) (SEQ ID NO: 1). An example of an NF-κB decoy is a decoy indicated by SEQ ID NO: 2, but is not limited thereto.

In another embodiment, it is possible to use a decoy comprising a sequence selected from among consensus sequences of the STAT-1 and STAT-3 binding sequence, TTCNNNGAA (N(A, G, T, C)) (SEQ ID NO: 3); consensus sequences of the STAT-6 binding sequence, TTCNNNNGAA (N(A, G, T, C)) (SEQ ID NO: 5); consensus sequences of the Ets binding sequence, MGGAW (M(A, C); W(A, T)) (SEQ ID NO: 7); consensus sequences of the AP-1 binding sequence, TGASTMA (S(G, C); M(A, C)) (SEQ ID NO: 9); consensus sequences of the E2F binding sequence, TTTSSCGS (S(G, C)) (SEQ ID NO: 11); consensus sequences of the Smad binding sequence, GTCT (SEQ ID NO: 13), CAGA (SEQ ID NO: 15), or GCCG (SEQ ID NO: 17); and the consensus sequence of the Runx2/Cbfa1 binding sequence, ACCACA (SEQ ID NO: 18).

Specific examples of the decoys that can be used include decoys comprising sequences selected from among the STAT-1 and STAT-3 binding sequence, TTCCGGGAA (SEQ ID NO: 4); the STAT-6 binding sequence, TTCCCAAGAA (SEQ ID NO: 6); the Ets binding sequence, CGGAA (SEQ ID NO: 8); the AP-1 binding sequence, TGAGTCA (SEQ ID NO: 10); the E2F binding sequence, TTTCCCGC (SEQ ID NO: 12); the Smad binding sequence, GTCTAGAC (SEQ ID NO: 14) or CAGACA (SEQ ID NO: 16); and the Runx2/Cbfa1 binding sequence, AACCACA (SEQ ID NO: 19), but are not limited thereto.

The decoys of the present invention may be composed of oligonucleotides. The oligonucleotides may be DNAs or RNAs, and they may comprise modified nucleic acids and/or pseudo nucleic acids. The oligonucleotides may be single stranded or double stranded, but are preferably double stranded. Mutants of these, compounds intramolecularly comprising these, or oligonucleotides comprising complements of these may also be used.

The decoys of the present invention can be produced using standard methods, such as by using DNA synthesizers.

The bone formation modulating agents of the present invention (for example, decoys) can be administered as pharmaceutical compositions locally, systemically, orally, or parenterally, alone or after mixing with common pharmaceutical carriers. For example, when inflammation is localized, as in rheumatoid arthritis, the agents can be injected locally Furthermore, for periodontal diseases such as alveolar pyorrhea, the agents can be injected into the gingival sulcus using an ointment as the base material.

The present pharmaceutical compositions can be provided in liquid dosage forms such as solutions, suspensions, syrups, liposomes, or lotions, or as solid dosage forms such as tablets, granules, powders, and capsules. If necessary, the pharmaceutical compositions may be supplemented with various vehicles, excipients, stabilizers, lubricants, and/or other conventional pharmaceutical additives.

The pharmaceutical compositions of the present invention can be used to prevent, ameliorate, and/or treat diseases caused by a disturbed balance between osteoclasts and osteoblasts, such as osteoporosis or Paget's disease. Alternatively, they can be used as preventive, ameliorating, and/or therapeutic agents for bone tumors, traumatic chondral defects, osteochondritis dissecans, osteoarthritis, rheumatoid arthritis, bone fractures, dislocations, periodontal diseases, and dental caries, as well as ameliorating agents for orthodontic and artificial dental implant treatments.

The doses of the decoys of the present invention differ depending on the age, body weight, symptoms, the methods of administration, administration routes, and such, but for intraarticular administration, intravascular administration, or intramuscular administration, for example, 10 nmol to 10,000 nmol can be administered to an adult per day at a single site or as divided in multiple sites.

Hereinbelow, the present invention will be specifically described with reference to Examples, but it is not to be construed as being limited thereto.

### Examples

### 1. Bone marrow cell culture and osteoclast differentiation

Bone marrow cells were prepared from three-day-old white rabbits. Bone marrow cells were taken out from rabbit femurs and tibias, collected into tubes, and washed twice with PBS. The fraction rich in mononucleated cells was separated from the bone marrow cells and cultured at 1 x 10⁵ cells/well using α-MEM medium supplemented with 10% fetal calf serum. Medium exchange was carried out every three days using α-MEM supplemented with vitamin D3 (1 x 10⁻⁸ M). In addition, luciferase gene (Clontech) regulated by an NF-κB binding sequence was transfected into the bone marrow cells using LipofectAMINE 2000 (Invitrogen). 24 hours after transfection, the cells were cultured for six hours in a serum-free medium. Resting state cells were treated with 1 x 10⁻⁸ M vitamin D3 for four hours or 48 hours, washed with PBS, and then lysed at 4°C for 15 minutes with 200 µL of cell lysis buffer. 20 µL of the cell extracts were mixed with 100 µL of Luciferase Assay Reagent and measured for two seconds each using a luminometer. Cells that were not treated with vitamin D3 but that were otherwise similarly treated were used as the control.

The following were used as the NF-κB decoys and scramble decoys. NF-κB decoy:
5'-CCTTGAAGGGATTTCCCTCC-3' (SEQ ID NO: 2)
3'-GGAACTTCCCTAAAGGGAGG-5'(SEQ ID NO: 21)
Scramble decoy:
5'-TTGCCGTACCTGACTTAGCC-3' (SEQ ID NO: 20)
3'-AACGGCATGGACTGAATCGG-5' (SEQ ID NO: 22)
The decoy oligonucleotides were prepared using a DNA synthesizer according to standard methods.

### 2. Tartrate-resistant acid phosphatase (TRAP) staining

Osteoclasts were detected by staining for TRAP, which is considered to be an osteoclast marker. Cells were treated with vitamin D3 (1 x 10⁻⁸ M) and scramble decoy or NF-κB decoy, then fixed on day 7 of culturing using 4.0% paraformaldehyde/PBS at room temperature for ten minutes. The cells were then TRAP stained (Code # OP04, Hokudo).

### 3. Nuclear staining using Hoechst 33258

Vitamin D3 (1×10⁻⁸ M) and scramble decoy or NF-κB decoy were added to the cells, which were then stained on day 7 of culturing using Hoechst 33258 (10 µL) for ten minutes. This was observed using a non-confocal microscope (Olympus BX60) at an excitation wavelength of 360 nm. (Since Hoechst 33258 stains all cell nuclei with a disrupted membrane, necrotic cells are observed as intact blue nuclei, while apoptotic cells are observed as fragmented (or condensed) nuclei.)

### 4. Observation by electron microscope

To store the cells and substrates, samples were fixed with 0.2 M cacodylate buffer supplemented with 5% glutaraldehyde overnight at 4°C and with 2% osmium for three hours. This was followed by stepwise dehydration of the samples using ethanol (70-100%). To use for scanning electron microscopy (SEM), samples were critical point dried using carbon dioxide. The samples were then coated with gold using a sputtering method, and observed by SEM (JEOL, T-200). To use for transmission electron microscopy (TEM), samples were dehydrated stepwise using ethanol (70-100%), and then embedded in Epok812. Ultra-thin sections were produced using an ultramicrotome (MT-7000, RMC), and these were placed on a collodion-coated copper grid. They were then stained using tannic acid, uranyl acetate, and lead citrate, and then observed by TEM (H-7000, Hitachi).

Results) After treating the cells with vitamin D3, TRAP-positive multinucleated cells were observed (see Fig. 1a, b, and c). Furthermore, cells treated with NF-κB decoy showed nuclear fragmentation (see Fig. 1d, e, and f).

Electron microscopic observations also showed that multinucleated cells covering the bone surface with a clear zone (Cz) are absorbed by the bone mineral through the ruffled borders (Rb). In Fig. 2(b), the upper part of the photograph shows an osteoclast, and the dark portion in the lower part of the photograph is a bone. Erosion of the bone by osteoclasts and formation of resorption pits (the lower left quarter or so of the photograph) can be seen. These osteoclasts moved along the surface of the bone and left small pits (see Figs. 2 and 13).

When luciferase gene regulated by an NF-κB binding sequence was transfected into the bone marrow cells of newborn rabbits to confirm NF-κB activation during osteoclast differentiation, luciferase activity was induced in cells treated with vitamin D3, 48 hours after treatment. This means that NF-κB activity increased (see Fig. 3 and Table 1).

**Table 1**

| NF-κB ACTIVITY | | (UNIT: RLU) |
|---|---|---|
| | MEAN | STANDARD ERROR |
| CONTROL | 2048.75 | 260. 1939981 |
| VITAMIN D3 (4 HOURS) | 1302 | 238. 092419 |
| VITAMIN D3 (48 HOURS) | 25899 | 3074. 668654 |

To confirm whether vitamin D3-induced NF-κB activity regulates both differentiation and activation of osteoclasts, NF-κB decoy was transfected into osteoclasts, and its inhibitory activity was investigated.

In cells transfected with FITC-labeled decoy (1 µM), activity was observed as a strong fluorescence in the nucleus one, four, and seven days after vitamin D3 treatment (see Fig. 4). Multinucleated cells were also observed by Hoechst 33258 nuclear staining. Fluorescence was detected up to 72 hours after transfection.

When luciferase gene regulated by an NF-κB sequence was transfected together with NF-κB decoy, luciferase activity decreased greatly in undifferentiated osteoclasts four days after vitamin D3 treatment (see Fig. 5 and Table 2, P < 0.01). By seven days after vitamin D3 treatment, luciferase activity had decreased greatly in differentiated osteoclasts (see Fig. 6 and Table 3, P < 0.01).

**Table 2**

| NF-κB ACTIVITY (FOUR DAYS AFTER VITAMIN D3 TREATMENT) | | (UNIT: RLU) |
|---|---|---|
| | MEAN | STANDARD ERROR |
| CONTROL | 2048.75 | 260.1939981 |
| VITAMIN D3 | 25899 | 3074. 668654 |
| VITAMIN D3 + NF-κB DECOY (0.5 µM) | 3338 | 798. 7757299 |
| VITAMIN D3 + NF-κB DECOY (1.0 µM) | 1375 | 906.9178574 |
| VITAMIN D3 + SCRAMBLE DECOY (1.0 µM) | 17612.5 | 2335.371134 |

**Table 3**

| NF-κB ACTIVITY (SEVEN DAYS AFTER VITAMIN D3 TREATMENT) | | (UNIT: RLU) |
|---|---|---|
| | MEAN | STANDARD ERROR |
| CONTROL | 3929.5 | 177. 5830791 |
| VITAMIN D3 | 31321 | 4318. 850985 |
| VITAMIN D3 + NF-κB DECOY (0. 5 µM) | 540. 25 | 88. 11391774 |
| VITAMIN D3 + NF-κB DECOY (1.0 µM) | 99 | 33. 96566894 |
| VITAMIN D3 + SCRAMBLE DECOY (1. 0 µM) | 21999 | 1859. 423656 |

Osteoclasts that differentiated from bone marrow cells due to vitamin D3 are detected as TRAP staining-positive multinucleated cells. TRAP-positive multinucleated osteoclasts significantly increased in the presence of vitamin D3 (see Fig. 7). However, when NF-κB decoy was transfected, the number of TRAP-positive cells greatly decreased compared to when scramble decoy was transfected (see Figs. 7 and 8, and Table 4, P<0.01). These treatments did not affect other mesenchymal osteoblastic cells.

**Table 4**

| TRAP-POSITIVE MULTINUCLEATED CELLS | | (UNIT: CELL NUMBER/FIELD) |
|---|---|---|
| | MEAN | STANDARD ERROR |
| VITAMIN D3 | 229.4666667 | 31.12616416 |
| VITAMIN D3 + NF-κB DECOY (0.25 µM) | 191.3333333 | 26.42149197 |
| VITAMIN D3 + NF-κB DECOY (0. 5 µM) | 62.6 | 22.33447048 |
| VITAMIN D3 + NF-κB DECOY (1. 0 µM) | 16.46666667 | 10.77607579 |
| VITAMIN D3 + SCRAMBLE DECOY (1.0 µM) | 209.4 | 26.58893433 |

Since NF-κB is well known as a factor that suppresses cell death, the effect of NF-κB decoy on osteoclast apoptosis was investigated. The cells underwent nuclear condensation, which is recognized as apoptosis (see Fig. 9). Cells that underwent apoptosis co-localize with the fluorescence of FITC-labeled NF-κB decoy.

Hence, induction of osteoclasts by vitamin D3 was inhibited by NF-κB decoy. This took place partly due to the induction of apoptosis.

TRAP staining was carried out with rat osteoclasts induced by M-CSF and RANKL. NF-κB decoy weakened induction of differentiation to rat osteoclasts by M-CSF and RANKL (see Fig. 10 and Table 5). From the above, as in the induction of differentiation of bone marrow cells to osteoclasts by vitamin D3, NF-κB was found to be involved in the induction of differentiation of bone marrow cells to osteoclasts by M-CSF and RANKL.

**Table 5**

| PERCENTAGE REDUCTION IN OSTEOCLAST FORMATION | | (UNIT: %) |
|---|---|---|
| | VITAMIN D3 (MEAN + STANDARD ERROR) | M-CSF + RANKL (MEAN + STANDARD ERROR) |
| NF-κB DECOY (0. 5 µM) | 63.5±11.2 | 71.2±7.3 |
| NF-κB DECOY (1. 0 µM) | 87.3±8.9 | 93.6±4.3 |

### 5. Observation of osteoclast formation

Osteoclasts were examined using a rat osteoclast formation kit (Hokudo, CAT# CUOCO1). Rat osteoclast precursor cells seeded in 24-well plates were incubated in a medium supplemented with M-CSF and RANKL. TRAP staining was performed seven days later.

### 6. Resorption pit formation

The effects of NF-κB decoy on resorption pit formation were examined. Three to four mm-thick dentine sections produced from human teeth were placed into 24-well plates. Bone marrow cells on the sixth day after vitamin D3 treatment (1×10⁴ cells/mL) were incubated for six hours on the dentine sections and then washed with PBS, then vitamin D3 (1 x 10⁻⁸ M) and NF-κB decoy or scramble decoy were further added, and this was cultured for three days. After removing the organic matter using sodium hypochlorite, the sections were stained with toluidine blue (SIGMA) and the resorption pits were visualized. Furthermore, the number of circular dots (osteoclast resorption pits) in the sections, which are reported to be related to osteoclast activity, were counted under a light microscope.

Results) TRAP staining was performed seven days after vitamin D3 treatment to measure osteoclast activity. The NF-κB decoy-administered dentine sections showed significant suppression of osteoclast activity compared to scramble decoy-administered dentine sections (see Figs. 11 and 12, and Table 6). NF-κB decoy can be said to have the effect of suppressing osteoclast activity by vitamin D3 in addition to suppressing induction by vitamin D3 of differentiation into osteoclasts.

**Table 6**

| NUMBER OF RESORPITON PITS IN DENTINE SECTIONS | | (UNIT: CELL NUMBER/FIELD) |
|---|---|---|
| | MEAN | STANDARD ERROR |
| SCRAMBLE DECOY (1.0 µM) | 992.5 | 105 |
| NF-κB DECOY (0. 25 µM) | 1125 | 154.1644 |
| NF-κB DECOY (0.5 µM) | 307.5 | 97.08244 |
| NF-κB DECOY (1.0 µM) | 23.75 | 17.01715 |

### 7. Rat osteoporosis model by ovariectomy

Female adult Wistar rats (ten weeks old) were purchased from Japan SLC (Shizuoka, Japan). After the rats were anesthetized with intraperitoneal ketamine (80 mg/kg) and xylazine (10 mg/kg), bilateral ovariectomies or sham operations (comparison subjects) were performed and osmotic mini-pumps (Alzet model 2004; Alza Corp) containing either NF-κB decoy or scramble decoy (infusion rate of 30 µg/kg/hour) were implanted. The body weights of these mice were recorded for two weeks. At two weeks after the operations, the mice were deeply anesthetized and their femurs, tibias, blood, and urine were collected for biochemical analyses. The proximal tibia and distal femur were cut out and homogenized in 10 mM triethanolamine buffer (pH 7.5), and supernatants were used to measure TRAP activity using the method of Walter (Walter, K. and Schutt, C., Acid and alkaline phosphatase in serum. Method of Enzymatic Analysis, Academic Press, New York & London: 1974; 856-870). The precipitates were hydrolyzed (hydized) for 24 hours at 105°C in hydrochloric acid (6 M), hydroxyprolines were measured by the method of Bergman, and the Ca content was measured based on the o-cresolphthalein complexone (OCPC) method by Gitelman (Gitelman, H. J., Kukolj, S., Welt, L. G. Inhibition of parathyroid gland activity by hypermagnesemia. Am. J. Physiol. 1968; 215:483-485). The estradiol level in the serum was measured by EIA (Mitsubishi Kagaku latron, Tokyo, Japan), and urinary deoxypyridinoline level was measured on day 14 of the experiments by EIA (Metra Biosystems, Mountain View, CA).

Results) The effects of NF-κB decoy on osteoporosis in the ovariectomized rat model

To further clarify the role of NF-κB in the activation of osteoclasts, the authors employed an estrogen-deficient ovariectomized rat model as a model of osteoporosis.

At 14 days after bilateral ovariectomy, serum estradiol levels were significantly decreased in the ovariectomized group, while there was no significant difference in body weight (Fig. 14a and Table 7). To confirm transfection into the bone, FITC-labeled oligonucleotides were administered using an osmotic mini-pump. As shown in Fig. 14b, fluorescence could be detected in the tibia. In a similar manner to the targeted cells, TRAP staining-positive cells were also detected after ovariectomy (Fig. 14c). In fact, TRAP activity was significantly increased in the tibia and femur of ovariectomized rats (Fig. 14d and Table 8).

In contrast, TRAP activity in the tibia and femur and the increase of TRAP staining-positive area were lowered by continuous treatment with NF-κB decoy using an osmotic mini-pump, but not with scramble decoy (Figs. 14c and 14d, and Table 8). Inhibition of the activation of osteoclasts was also confirmed by the measurement of calcium and hydroxyproline, which are typical markers for tissue collagen. As shown in Figs. 15a and 15b, the concentrations of calcium and hydroxyproline in the tibia and femur were decreased after ovariectomy, but the decrease was significantly attenuated by NF-κB decoy treatment (P < 0.01; Fig. 15a, Table 9; and Fig. 15b, Table 10).

These results were accompanied with changes in urinary deoxypyridinoline released from the bone by the processing of tissue collagen. Treatment with NF-κB decoy suppressed the ovariectomy-induced increase in urinary deoxypyridinoline (P< 0.01, Fig. 15c and Table 11). These results suggest that treatment with NF-κB decoy ameliorates estrogen deficiency-induced osteoporosis.

**Table 7**

| BODY WEIGHT AND ESTRADIOL CONCENTRATION | | |
|---|---|---|
| | BODY WEIGHT (g) | ESTRADIOL (pg/ml) |
| Sham | 230.5±7.1 | 29.6±2.4 |
| OVX | 232.1±3.6 | 20.3±1.8 |
| NF-κB | 228.8±6.3 | 20.5±3.5 |
| Scb | 232.1±16.6 | 18.5±0.5 |

**Table 8**

| LEVEL OF TRAP POSITIVITY | | (UNIT: U/BONE) |
|---|---|---|
| | TIBIA | FEMUR |
| Sham | 0.208±0.005 | 0.204±0.004 |
| OVX | 0.264±0.014 | 0.232±0.007 |
| NF-κB | 0.224±0.029 | 0.181±0.010 |
| Scb | 0.258±0.018 | 0.192±0.016 |

**Table 9**

| CALCIUM CONCENTRATION | | (UNIT: mg/BONE) |
|---|---|---|
| | TIBIA | FEMUR |
| Sham | 20.73±0.69 | 24. 89±1. 55 |
| OVX | 16.94±0.70 | 15.8±0.79 |
| NF-κB | 18.58±1.10 | 21.09±0.22 |
| Scb | 17.20±0.47 | 15.84±1. 71 |

**Table 10**

| HYDROXYPROLINE CONCENTRATION | | (UNIT: µmol/BONE) |
|---|---|---|
| | TIBIA | FEMUR |
| Sham | 22.94±1.03 | 23.93±0.57 |
| OVX | 15.22±1.01 | 18.78±0.06 |
| NF-κB | 22.65±1.32 | 20.00±0.63 |
| Scb | 14.19±1.10 | 18.55±0.86 |

### 8. Vitamin C-deficient rat model

Osteogenic disorder Shionogi (ODS) rats are a mutant strain of Wistar rats that are deficient in a key enzyme for ascorbate synthesis, in which several hydroxylases such as prolinehydroxylase are decreased (Togari, A., Arai, M., Nakagawa, S., Banno, A., Aoki, M., Matsumoto S., Alteration of bone status with ascorbic acid deficiency in ODS (osteogenic disorder Shionogi) rat, Jpn. J. Pharmacol., 1995 Jul; 68(3):255-261). When these rats are raised normally on a vitamin C-supplemented diet until they are nine weeks old and then given a deficient diet, collagen synthesis cannot be carried out, causing disintegration of blood vessels and bones and leading to death in approximately five weeks.

Male osteogenic disorder Shionogi rats (eight weeks old) were purchased from CLEA laboratory and fed a diet supplemented with L-ascorbate at 800 mg/kg body weight. One week later, the diet was changed to an L-ascorbate-deficient diet, and osmotic mini-pumps containing either NF-κB or scramble decoy ODN (infusion rate of 30 µg/kg/hour) were implanted in the rats. The body weight of these rats was recorded before and on the fourth week after terminating L-ascorbate administration. The tibias and femurs were analyzed on the fourth week after the operation.

### 9. Dual energy X-ray absorptiometry (DXA)

Bone density (BMD) (g/cm²) was measured using a bone density X-ray apparatus, the dual-photon X-ray absorptiometry (DEXA) bone densitometer (GE-Lunar DPX-IQ, Madison, WI). High- and low-beam energies for all scans were 80 and 35 kV respectively, at 0.5 mA, as described by Venken, K. et al., Bone 2005, 36:663-670.

Results) Effects of NF-κB decoy ODN on osteoporosis in a vitamin C-deficient rat model

Osteogenic disorder Shionogi rats, which are deficient in a key enzyme for ascorbate synthesis, were used to further verify the therapeutic effect of NF-κB decoy in osteoporosis.

After termination of the L-ascorbate-supplemented diet, the body weight of the osteogenic disorder Shionogi rats dramatically decreased and bone length was shortened. However, NF-κB decoy ODN treatment inhibited the decrease in body weight (Fig. 16a). Furthermore, there were significant differences in the length and weight of the femur. (Length of femur: control, 36.8 ± 0.2 mm; NF-κB decoy, 32.6 ± 0.2 mm*; scramble decoy, 30.1 ± 0.5 mm; *P < 0.05 compared to scramble decoy Weight of femur: control; 0.866 ± 0.01 g; NF-κB decoy, 0.639 ± 0.019 g*; scramble decoy, 0.557 ± 0.010 g; *P < 0.05 compared to scramble decoy.)

Bone fracture was frequently found in the proximal femur of the groups treated with scramble decoy ODN, but not in the groups treated with NF-κB decoy ODN. As shown in Fig. 16b, the femoral head (indicated by arrows) was not decayed and was preserved only in NK-κB decoy ODN-treated groups. Importantly, bone density analysis by dual-energy X-ray absorptiometry showed a significant increase in bone density at the distal femur (the knee joint portion) in NF-κB decoy ODN-treated groups (Fig. 16c). These results suggest that NF-κB decoy ODN treatment ameliorates osteopororic changes in osteogenic disorder Shionogi rats.

### 10. Statistical analyses

All values were indicated as a mean ± standard error. Variance was determined by the Bonferroni/Dunnett method, and significance was examined for numerous comparisons. P < 0.05 was considered as a significant difference.

### Industrial Applicability

The present invention provides preventive, ameliorating, and/or therapeutic agents for diseases caused by a disturbed balance between bone formation and bone resorption.

## Claims

1. A bone formation modulating agent, in which a nucleic acid comprising a bone formation-related transcriptional regulatory factor binding sequence is an active ingredient.

2. The bone formation modulating agent of claim 1, wherein the nucleic acid is selected from a group consisting of a decoy, an antisense, a ribozyme, an aptamer, and an siRNA.

3. The bone formation modulating agent of claim 1, wherein the nucleic acid is a decoy comprising a DNA or an RNA.

4. The bone formation modulating agent of claim 2 or 3, wherein the decoy comprises a DNA oligonucleotide.

5. The bone formation modulating agent of any one of claims 2 to 4, wherein the decoy is a decoy of NF-κB, STAT-1, STAT-3, STAT-6, Ets, AP-1, E2F, Smad1, Smad2, Smad3, Smad4, Smad5, Smad6, Smad7, Smad8, or Runx2/Cbfa1.

6. The bone formation modulating agent of claim 5, wherein the decoy is a decoy of NF-κB.

7. The bone formation modulating agent of any one of claims 2 to 6, wherein the decoy comprises the NF-κB binding sequence GGGRHTYYHC (wherein R means A or G, Y means C or T, and H means A, C, or T).

8. The bone formation modulating agent of claim 7, wherein the decoy comprises the NF-κB binding sequence GGGATTTCCC (SEQ ID NO: 23) or GGGACTTTCC (SEQ ID NO: 24).

9. The bone formation modulating agent of any one of claims 2 to 8, wherein the decoy is a decoy represented by SEQ ID NO: 2.

10. The bone formation modulating agent of any one of claims 2 to 5, wherein the decoy comprises the STAT-1 or STAT-3 binding sequence TTCNNNGAA (wherein N means A, G, T, or C).

11. The bone formation modulating agent of claim 10, wherein the decoy comprises the STAT-1 or STAT-3 binding sequence TTCCGGGAA.

12. The bone formation modulating agent of any one of claims 2 to 5, wherein the decoy comprises the STAT-6 binding sequence TTCNNNNGAA (wherein N means A, G, T, or C).

13. The bone formation modulating agent of claim 12, wherein the decoy comprises the STAT-6 binding sequence TTCCCAAGAA.

14. The bone formation modulating agent of any one of claims 2 to 5, wherein the decoy comprises the Ets binding sequence MGGAW (wherein M means A or C, and W means A or T).

15. The bone formation modulating agent of claim 14, wherein the decoy comprises the Ets binding sequence CGGAA.

16. The bone formation modulating agent of any one of claims 2 to 5, wherein the decoy comprises the AP-1 binding sequence TGASTMA (wherein S means G or C, and M means A or C).

17. The bone formation modulating agent of claim 16, wherein the decoy comprises the AP-1 binding sequence TGAGTCA.

18. The bone formation modulating agent of any one of claims 2 to 5, wherein the decoy comprises the E2F binding sequence TTTSSCGS (wherein S means G or C).

19. The bone formation modulating agent of claim 18, wherein the decoy comprises the E2F binding sequence TTTCCCGC.

20. The bone formation modulating agent of any one of claims 2 to 5, wherein the decoy comprises the Smad1, Smad2, Smad3, Smad4, Smad5, Smad6, Smad7, or Smad8 binding sequence GTCT, CAGA, or GCCG

21. The bone formation modulating agent of claim 20, wherein the decoy comprises the Smad1, Smad2, Smad3, Smad4, Smad5, Smad6, Smad7, or Smad8 binding sequence GTCTAGAC or CAGACA.

22. The bone formation modulating agent of any one of claims 2 to 5, wherein the decoy comprises the Ruux2/Cbfa1 binding sequence ACCACA.

23. The bone formation modulating agent of claim 22, wherein the decoy comprises the Runx2/Cbfa1 binding sequence AACCACA.

24. The bone formation modulating agent of any one of claims 2 to 23, wherein the decoy comprises a double-stranded DNA.

25. A preventive agent, ameliorating agent, or therapeutic agent for a disease caused by a disturbed balance between osteoclasts and osteoblasts, wherein the agent comprises the bone formation modulating agent of any one of claims 1 to 24.

26. A preventive agent, ameliorating agent, or therapeutic agent, wherein the disease of claim 25 is any one selected from osteoporosis, Paget's disease, bone tumor, traumatic chondral defect, osteochondritis dissecans, osteoarthritis, rheumatoid arthritis, bone fracture, dislocation, periodontal disease, and dental caries.

27. An ameliorating agent for an orthodontic or artificial dental implant treatment, which comprises the bone formation modulating agent of any one of claims 1 to 24.

28. A method for modulating bone formation that uses a nucleic acid comprising a bone formation-related transcriptional regulatory factor binding sequence.

29. The method of claim 28, wherein the nucleic acid is selected from a group consisting of a decoy, an antisense, a ribozyme, an aptamer, and an siRNA.

30. The method of claim 28, wherein the nucleic acid is a decoy comprising a DNA or an RNA.

31. The method of claim 29 or 30, wherein the decoy comprises a DNA oligonucleotide.

32. The method of any one of claims 29 to 31, wherein the decoy is a decoy of NF-κB, STAT-1, STAT-3, STAT-6, Ets, AP-1, E2F, Smad1, Smad2, Smad3, Smad4, Smad5, Smad6, Smad7, Smad8, or Runx2/Cbfa1.

33. The method of claim 32, wherein the decoy is a decoy of NF-κB.

34. The method of any one of claims 29 to 33, wherein the decoy comprises the NF-κB binding sequence GGGRHTYYHC (wherein R means A or G, Y means C or T, and H means A, C, or T).

35. The method of claim 34, wherein the decoy comprises the NF-κB binding sequence GGGATTTCCC (SEQ ID NO: 23) or GGGACTTTCC (SEQ ID NO: 24).

36. The method of any one of claims 29 to 35, wherein the decoy is a decoy represented by SEQ ID NO: 2.

37. The method of any one of claims 29 to 32, wherein the decoy comprises the STAT-1 or STAT-3 binding sequence TTCNNNGAA (wherein N means A, G, T, or C).

38. The method of claim 37, wherein the decoy comprises the STAT-1 or STAT-3 binding sequence TTCCGGGAA.

39. The method of any one of claims 29 to 32, wherein the decoy comprises the STAT-6 binding sequence TTCNNNNGAA (wherein N means A, G, T, or C).

40. The method of claim 39, wherein the decoy comprises the STAT-6 binding sequence TTCCCAAGAA.

41. The method of any one of claims 29 to 32, wherein the decoy comprises the Ets binding sequence MGGAW (wherein M means A or C, and W means A or T).

42. The method of claim 41, wherein the decoy comprises the Ets binding sequence CGGAA.

43. The method of any one of claims 29 to 32, wherein the decoy comprises the AP-1 binding sequence TGASTMA (wherein S means G or C, and M means A or C).

44. The method of claim 43, wherein the decoy comprises the AP-1 binding sequence TGAGTCA.

45. The method of any one of claims 29 to 32, wherein the decoy comprises the E2F binding sequence TTTSSCGS (wherein S means G or C).

46. The method of claim 45, wherein the decoy comprises the E2F binding sequence TTTCCCGC.

47. The method of any one of claims 29 to 32, wherein the decoy comprises the Smad1, Smad2, Smad3, Smad4, Smad5, Smad6, Smad7, or Smad8 binding sequence GTCT, CAGA, or GCCG.

48. The method of claim 47, wherein the decoy comprises the Smad1, Smad2, Smad3, Smad4, Smad5, Smad6, Smad7, or Smad8 binding sequence GTCTAGAC or CAGACA.

49. The method of any one of claims 29 to 32, wherein the decoy comprises the Runx2/Cbfa1 binding sequence ACCACA.

50. The method of claim 49, wherein the decoy comprises the Runx2/Cbfa1 binding sequence AACCACA.

51. The method of any one of claims 29 to 50, wherein the decoy comprises a double-stranded DNA.

52. The method of any one of claims 28 to 51 for preventing, ameliorating, or treating a disease caused by a disturbed balance between osteoclasts and osteoblasts.

53. The method of claim 52, wherein the disease caused by the disturbed balance between osteoclasts and osteoblasts is selected from the group consisting of osteoporosis, Paget's disease, bone tumor, traumatic chondral defect, osteochondritis dissecans, osteoarthritis, rheumatoid arthritis, bone fracture, dislocation, periodontal disease, and dental caries.

54. The method of any one of claims 28 to 51 for ameliorating an orthodontic or artificial dental implant treatment.

55. A use of a nucleic acid comprising a bone formation-related transcriptional regulatory factor binding sequence for producing a pharmaceutical agent for modulating bone formation.

56. The use of claim 55, wherein the nucleic acid is selected from a group consisting of a decoy, an antisense, a ribozyme, an aptamer, and an siRNA.

57. The use of claim 55, wherein the nucleic acid is a decoy comprising a DNA or an RNA.

58. The use of claim 56 or 57, wherein the decoy comprises a DNA oligonucleotide.

59. The use of any one of claims 56 to 58, wherein the decoy is a decoy of NF-κB, STAT-1, STAT-3, STAT-6, Ets, AP-1, E2F, Smad1, Smad2, Smad3, Smad4, Smad5, Smad6, Smad7, Smad8, or Runx2/Cbfa1.

60. The use of claim 59, wherein the decoy is a decoy of NF-κB.

61. The use of any one of claims 56 to 60, wherein the decoy comprises the NF-κB binding sequence GGGRHTYYHC (wherein R means A or G, Y means C or T, and H means A, C, or T).

62. The use of claim 61, wherein the decoy comprises the NF-κB binding sequence GGGATTTCCC (SEQ ID NO: 23) or GGGACTTTCC (SEQ ID NO: 24).

63. The use of any one of claims 56 to 62, wherein the decoy is a decoy represented by SEQ ID NO: 2.

64. The use of any one of claims 56 to 59, wherein the decoy comprises the STAT-1 or STAT-3 binding sequence TTCNNNGAA (wherein N means A, G, T, or C).

65. The use of claim 64, wherein the decoy comprises the STAT-1 or STAT-3 binding sequence TTCCGGGAA.

66. The use of any one of claims 56 to 59, wherein the decoy comprises the STAT-6 binding sequence TTCNNNNGAA (wherein N means A, G, T, or C).

67. The use of claim 66, wherein the decoy comprises the STAT-6 binding sequence TTCCCAAGAA.

68. The use of any one of claims 56 to 59, wherein the decoy comprises the Ets binding sequence MGGAW (wherein M means A or C, and W means A or T).

69. The use of claim 68, wherein the decoy comprises the Ets binding sequence CGGAA.

70. The use of any one of claims 56 to 59, wherein the decoy comprises the AP-1 binding sequence TGASTMA (wherein S means G or C, and M means A or C).

71. The use of claim 70, wherein the decoy comprises the AP-1 binding sequence TGAGTCA.

72. The use of any one of claims 56 to 59, wherein the decoy comprises the E2F binding sequence TTTSSCGS (wherein S means G or C).

73. The use of claim 72, wherein the decoy comprises the E2F binding sequence TTTCCCGC.

74. The use of any one of claims 56 to 59, wherein the decoy comprises the Smad1, Smad2, Smad3, Smad4, Smad5, Smad6, Smad7, or Smad8 binding sequence GTCT, CAGA, or GCCG

75. The use of claim 74, wherein the decoy comprises the Smad1, Smad2, Smad3, Smad4, Smad5, Smad6, Smad7, or Smad8 binding sequence GTCTAGAC or CAGACA.

76. The use of any one of claims 56 to 59, wherein the decoy comprises the Runx2/Cbfa1 binding sequence ACCACA.

77. The use of claim 76, wherein the decoy comprises the Runx2/Cbfa1 binding sequence AACCACA.

78. The use of any one of claims 56 to 77, wherein the decoy comprises a double-stranded DNA.

79. The use of any one of claims 55 to 78, wherein the bone formation modulating agent prevents, ameliorates, or treats a disease caused by a disturbed balance between osteoclasts and osteoblasts.

80. The use of claim 79, wherein the disease caused by a disturbed balance between osteoclasts and osteoblasts is selected from the group consisting of osteoporosis, Paget's disease, bone tumor, traumatic chondral defect, osteochondritis dissecans, osteoarthritis, rheumatoid arthritis, bone fracture, dislocation, periodontal disease, and dental caries.

81. The use of any one of claims 55 to 78, wherein the bone formation modulating agent ameliorates an orthodontic or artificial dental implant treatment.
